# EUROPEAN PATENT SPECIFICATION

(11) **EP 1 252 317 B1**
(45) Date of publication and mention of the grant of the patent: **04.07.2007**
(21) Application number: 01908833.5
(22) Date of filing: 02.02.2001
(51) Int. Cl.: C12N 15/54, C12N 9/10

(54) **HUMAN ENZYMES AND POLYNUCLEOTIDES ENCODING THE SAME**
MENSCHLICHE ENZYME UND DAFÜR KODIERENDE POLYNUKLEOTIDE
NOUVEAUX ENZYMES HUMAINS ET POLYNUCLEOTIDES LES CODANT

(30) Priority: 04.02.2000 US 180413 P
(43) Date of publication of application: 30.10.2002
(73) Proprietor: Lexicon Genetics Incorporated, The Woodlands, TX 77381-1160 (US)
(72) Inventor: WALKE, D., Wade, Spring, TX 77389 (US); WILGANOWSKI, Nathaniel, L., Spring, TX 77388 (US); TURNER, C., Alexander, Jr., The Woodlands, TX 77382 (US); FRIEDRICH, Glenn, c/o BCM Technologies, Houston, TX 77046 (US); ABUIN, Alejandro, The Woodlands, TX 77382 (US); ZAMBROWICZ, Brian, The Woodlands, TX 77382 (US); SANDS, Arthur, T., The Woodlands, TX 77381 (US)
(74) Representative: Silveston, Judith
(86) International application number: PCT/US2001/003652
(87) International publication number: WO 2001/057220

(56) References cited:
- WO-A-91/11513
- HATTORI ET AL.: "Homo sapiens genomic DNA, chromosome 11q13, clone:RP11-807H22" EMBL SEQUENCE DATABASE, 3 December 1999 (1999-12-03), XP002172248 HEIDELBERG DE
- NCI-CGAP: "on98a03.s1 Soares_NFL_T_GBC_S1 Homo sapiens cDNA clone IMAGE:1564684 3' similar to SW:COMT_PIG Q99028 CATECHOL-O-METHYLTRANSFERASE, SOLUBLE FORM" EMBL SEQUENCE DATABASE, 27 April 1998 (1998-04-27), XP002172249 HEIDELBERG DE
- FELDMAN AND HENDERSON: "Mono amine oxidase EC-1.4.3.4 catechol-o-methyl transferase EC-2.2.2.6 and norepinephrine levels in mice with the hereditary obese hyper glycemic syndrome." DIABETES, vol. 27, no. 4, 1978, pages 389-395,

## Description

### 1. INTRODUCTION

The present invention relates to the discovery, identification, and characterization of novel human polynucleotides encoding proteins sharing sequence similarity with mammalian enzymes. The invention encompasses the described polynucleotides, host cell expression systems, the encoded proteins, fusion proteins and polypeptides and peptides. Also disclosed are antibodies to the encoded proteins and peptides. The invention encompasses genetically engineered non-human animals that over express the disclosed sequences, and discloses antagonists and agonists of the proteins, and other compounds that modulate the expression or activity of the proteins encoded by the disclosed polynucleotides that can be used for diagnosis, drug screening, clinical trial monitoring and the treatment of physiological disorders.

### 2. BACKGROUND OF THE INVENTION

Enzymes are biological catalysts that modify biological substrates, including proteins, as part of degradation, maturation, catabolic, metabolic, differentiation, and secretory pathways within the body. Enzyme abnormalities have thus been associated with, *inter alia,* growth, development, protein and cellular senescence, cancer, and other diseases. WO 91/11513 discloses a method of isolating from rat liver and human placenta catechol-O-methyltransferase (EC 2.1.1.6, COMT) enzyme to a degree sufficient to allow amino acid sequencing of the enzyme and identification of recombinant clones directed to the enzyme.

The EMBL sequence database discloses, under accession number AP000817 the Homo sapiens genomic sequence of a clone derived from chromosome 11q (clone RP11-807H22). Under accession number AA928744 the sequence of a Homo sapiens cDNA clone identified as Soares_NFL_T_GBC_S1 Homo sapiens cDNA clone IMAGE: 1564684 3' similar to SW: COMT_PIG Q99028 CATECHOL-O-METHYLTRANSFERASE, SOLUBLE FORM is disclosed.

### 3. SUMMARY OF THE INVENTION

The present invention relates to the discovery, identification, and characterization of nucleotides that encode novel human proteins, and the corresponding amino acid sequences of these proteins. The novel human proteins (NHPs) described for the first time herein share structural similarity with methyltransferase proteins, and particularly catechol-O-methyltransferases from a variety of organisms.

The novel human nucleic acid (cDNA) sequences described herein encode proteins/open reading frames (ORFs) of 258 and 168 amino acids in length (see respectively SEQ ID NOS: 2 and 4).

The invention also relates to agonists and antagonists of the described NHPs including small molecules, large molecules, mutant NHPs, or portions thereof that compete with native NHPs, NHP peptides, and antibodies, as well as nucleotide sequences that can be used to inhibit the expression of the described NHPs (*e.g.,* antisense and ribozyme molecules, and gene or regulatory sequence replacement constructs) or to enhance the expression of the described NHPs (e.g., expression constructs that place the described sequence under the control of a strong promoter system). The invention encompasses non-human transgenic animals that express a NHP transgene. A gene trapped knockout ES cell line has been produced in a murine ortholog of the disclosed NHPs.

Further, the present invention also relates to processes for identifying compounds that modulate, i.e., act as agonists or antagonists, of NHP expression and/or NHP activity that utilize purified preparations of the described NHP and/or NHP product, or cells expressing the same. Such compounds can be used as therapeutic agents for the treatment of any of a wide variety of symptoms associated with biological disorders or imbalances.

### 4. DESCRIPTION OF THE SEQUENCE LISTING AND FIGURES

The Sequence Listing provides the sequences of the NHP ORFs encoding the described NHP amino acid sequences. SEQ ID NO:5 describes a NHP ORF with flanking sequences.

### 5. DETAILED DESCRIPTION OF THE INVENTION

The NHPs described for the first time herein, are novel proteins that are expressed in, *inter alia,* human cell lines, gene trapped cells, and human fetal brain and testis cells.

The described sequences were compiled from gene trapped cDNAs and clones isolated from human testis and brain cDNA libraries (Edge Biosystems, Gaithersburg, MD). The present invention encompasses the nucleotides presented in the Sequence Listing, host cells expressing such nucleotides, the expression products of such nucleotides, and: (a) nucleotides that encode mammalian homologs of the described sequences, including the specifically described NHPs, and the NHP products; (b) nucleotides that encode one or more portions of a NHP that correspond to functional domains of the NHP, and the polypeptide products specified by such nucleotide sequences, including but not limited to the novel regions of any active domain(s); (c) isolated nucleotides that encode mutant versions, engineered or naturally occurring, of a described NHP in which all or a part of at least one domain is deleted or altered, and the polypeptide products specified by such nucleotide sequences, including but not limited to soluble proteins and peptides in which all or a portion of the signal sequence is deleted; (d) nucleotides that encode chimeric fusion proteins containing all or a portion of a coding region of a NHP, or one of its domains (e.g., a receptor or ligand binding domain, accessory protein/self-association domain, etc.) fused to another peptide or polypeptide; or (e) therapeutic or diagnostic derivatives of the described polynucleotides such as oligonucleotides, antisense polynucleotides, ribozymes, dsRNA, or gene therapy constructs comprising a sequence first disclosed in the Sequence Listing. As discussed above, the present invention includes: (a) the human DNA sequences presented in the Sequence Listing (and vectors comprising the same) and additionally contemplates any nucleotide sequence encoding a contiguous NHP open reading frame (ORF), or a contiguous exon splice junction first described in the Sequence Listing, that hybridizes to a complement of a DNA sequence presented in the Sequence Listing under highly stringent conditions, e.g., hybridization to filter-bound DNA in 0.5 M NaHPO₄, 7% sodium dodecyl sulfate (SDS), 1 mM EDTA at 65°C, and washing in 0.1xSSC/0.1% SDS at 68°C (Ausubel F.M. et al., eds., 1989, Current Protocols in Molecular Biology, Vol. I, Green Publishing Associates, Inc., and John Wiley & sons, Inc., New York, at p. 2.10.3) and encodes a functionally equivalent gene product. Additionally contemplated are any nucleotide sequences that hybridize to the complement of the DNA sequence that encode and express an amino acid sequence presented in the Sequence Listing under moderately stringent conditions, e.g., washing in 0.2xSSC/0.1% SDS at 42°C (Ausubel et al., 1989, *supra*), yet still encode a functionally equivalent NHP product. Functional equivalents of a NHP include naturally occurring NHPs present in other species and mutant NHPs whether naturally occurring or engineered (by site directed mutagenesis, gene shuffling, directed evolution as described in, for example, U.S. Patent No. 5,837,458). The invention also includes degenerate nucleic acid variants of the disclosed NHP polynucleotide sequences.

Additionally contemplated are polynucleotides encoding a NHP ORF, or its functional equivalent, encoded by a polynucleotide sequence that is about 99, 95, 90, or about 85 percent similar or identical to corresponding regions of the nucleotide sequences of the Sequence Listing (as measured by BLAST sequence comparison analysis using, for example, the GCG sequence analysis package using standard default settings).

The invention also includes nucleic acid molecules, preferably DNA molecules, that hybridize to, and are therefore the complements of, the described NHP nucleotide sequences. Such hybridization conditions may be highly stringent or less highly stringent, as described above. In instances where the nucleic acid molecules are deoxyoligonucleotides ("DNA oligos"), such molecules are generally about 16 to about 100 bases long, or about 20 to about 80, or about 34 to about 45 bases long, or any variation or combination of sizes represented therein that incorporate a contiguous region of sequence first disclosed in the Sequence Listing. Such oligonucleotides can be used in conjunction with the polymerase chain reaction (PCR) to screen libraries, isolate clones, and prepare cloning and sequencing templates, etc.

Alternatively, such NHP oligonucleotides can be used as hybridization probes for screening libraries, and assessing gene expression patterns (particularly using a micro array or high-throughput "chip" format). Additionally, a series of the described NHP oligonucleotide sequences, or the complements thereof, can be used to represent all or a portion of the described NHP sequences. An oligonucleotide or polynucleotide sequence first disclosed in at least a portion of one or more of the sequences of SEQ ID NOS: 1-5 can be used as a hybridization probe in conjunction with a solid support matrix/substrate (resins, beads, membranes, plastics, polymers, metal or metallized substrates, crystalline or polycrystalline substrates, etc.). Of particular note are spatially addressable arrays (i.e., gene chips, microtiter plates, etc.) of oligonucleotides and polynucleotides, or corresponding oligopeptides and polypeptides, wherein at least one of the biopolymers present on the spatially addressable array comprises an oligonucleotide or polynucleotide sequence first disclosed in at least one of the sequences of SEQ ID NOS: 1-5, or an amino acid sequence encoded thereby. Methods for attaching biopolymers to, or synthesizing biopolymers on, solid support matrices, and conducting binding studies thereon are disclosed in, *inter* alia, U.S. Patent Nos. 5,700,637, 5,556,752, 5,744,305, 4,631,211, 5,445,934, 5,252,743, 9,713,326, 5,424,186, and 4,689,405.

Addressable arrays comprising sequences first disclosed in SEQ ID NOS:1-5 can be used to identify and characterize the temporal and tissue specific expression of a gene. These addressable arrays incorporate oligonucleotide sequences of sufficient length to confer the required specificity, yet be within the limitations of the production technology. The length of these probes is within a range of between about 8 to about 2000 nucleotides. Preferably the probes consist of 60 nucleotides, and more preferably 25 nucleotides, from the sequences first disclosed in SEQ ID NOS:1-5.

For example, a series of the described oligonucleotide sequences, or the complements thereof, can be used in chip format to represent all or a portion of the described sequences. The oligonucleotides, typically between about 16 to about 40 (or any whole number within the stated range) nucleotides in length can partially overlap each other and/or the sequence may be represented using oligonucleotides that do not overlap. Accordingly, the described polynucleotide sequences shall typically comprise at least about two or three distinct oligonucleotide sequences of at least about 8 nucleotides in length that are each first disclosed in the described Sequence Listing. Such oligonucleotide sequences can begin at any nucleotide present within a sequence in the Sequence Listing and proceed in either a sense (5'-to-3') orientation vis-a-vis the described sequence or in an antisense orientation.

Microarray-based-analysis allows the discovery of broad patterns of genetic activity, providing new understanding of gene functions and generating novel and unexpected insight into transcriptional processes and biological mechanisms. The use of addressable arrays comprising sequences first disclosed in SEQ ID NOS:1-5 provides detailed information about transcriptional changes involved in a specific pathway, potentially leading to the identification of novel components or gene functions that manifest themselves as novel phenotypes.

Probes consisting of sequences first disclosed in SEQ ID NOS:1-5 can also be used in the identification, selection and validation of novel molecular targets for drug discovery. The use of these unique sequences permits the direct confirmation of drug targets and recognition of drug dependent changes in gene expression that are modulated through pathways distinct from the drugs intended target. These unique sequences therefore also have utility in defining and monitoring both drug action and toxicity.

As an example of utility, the sequences first disclosed in SEQ ID NOS:1-5 can be utilized in microarrays or other assay formats, to screen collections of genetic material from patients who have a particular medical condition. These investigations can also be carried out using the sequences first disclosed in SEQ ID NOS:1-5 *in silico* and by comparing previously collected genetic databases and the disclosed sequences using computer software known to those in the art.

Thus, the sequences first disclosed in SEQ ID NOS:1-5 can be used to identify mutations associated with a particular disease and also as a diagnostic or prognostic assay.

Although the presently described sequences have been specifically described using nucleotide sequence, it should be appreciated that each of the sequences can uniquely be described using any of a wide variety of additional structural attributes, or combinations thereof. For example, a given sequence can be described by the net composition of the nucleotides present within a given region of the sequence in conjunction with the presence of one or more specific oligonucleotide sequence(s) first disclosed in the SEQ ID NOS: 1-5. Alternatively, a restriction map specifying the relative positions of restriction endonuclease digestion sites, or various palindromic or other specific oligonucleotide sequences can be used to structurally describe a given sequence. Such restriction maps, which are typically generated by widely available computer programs (e.g., the University of Wisconsin GCG sequence analysis package, SEQUENCHER 3.0, Gene Codes Corp., Ann Arbor, MI, etc.), can optionally be used in conjunction with one or more discrete nucleotide sequence(s) present in the sequence that can be described by the relative position of the sequence relatve to one or more additional sequence(s) or one or more restriction sites present in the disclosed sequence.

For oligonucleotide probes, highly stringent conditions may refer, for example, to washing in 6xSSC/0.05% sodium pyrophosphate at 37°C (for 14-base oligos), 48°C (for 17-base oligos), 55°C (for 20-base oligos), and 60°C (for 23-base oligos). These nucleic acid molecules may encode or act as NHP gene antisense molecules, useful, for example, in NHP gene regulation (for and/or as antisense primers in amplification reactions of NHP gene nucleic acid sequences). With respect to NHP gene regulation, such techniques can be used to regulate biological functions. Further, such sequences may be used as part of ribozyme and/or triple helix sequences that are also useful for NHP gene regulation.

Inhibitory antisense or double stranded oligonucleotides can additionally comprise at least one modified base moiety which is selected from the group including but not limited to 5-fluorouracil, 5-bromouracil, 5-chlorouracil, 5-iodouracil, hypoxanthine, xantine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine.

The antisense oligonucleotide can also comprise at least one modified sugar moiety selected from the group, including but not limited to, arabinose, 2-fluoroarabinose, xylulose, and hexose.

In yet another embodiment, the antisense oligonucleotide will comprise at least one modified phosphate backbone selected from the group consisting of a phosphorothioate, a phosphorodithioate, a phosphoramidothioate, a phosphoramidate, a phosphordiamidate, a methylphosphonate, an alkyl phosphotriester, and a formacetal or analog thereof.

In yet another embodiment, the antisense oligonucleotide is an α-anomeric oligonucleotide. An α-anomeric oligonucleotide forms specific double-stranded hybrids with complementary RNA in which, contrary to the usual β-units, the strands run parallel to each other (Gautier et al., 1987, Nucl. Acids Res. 15:6625-6641). The oligonucleotide is a 2*'*-0-methylribonucleotide (Inoue et al., 1987, Nucl. Acids Res. 15:6131-6148), or a chimeric RNA-DNA analogue (Inoue et al., 1987, FEBS Lett. 215:327-330). Alternatively, double stranded RNA can be used to disrupt the expression and function of a targeted NHP.

Oligonucleotides of the invention can be synthesized by standard methods known in the art, e.g. by use of an automated DNA synthesizer (such as are commercially available from Biosearch, Applied Biosystems, etc.). As examples, phosphorothioate oligonucleotides can be synthesized by the method of Stein et al. (1988, Nucl. Acids Res. 16:3209), and methylphosphonate oligonucleotides can be prepared by use of controlled pore glass polymer supports (Sarin et al., 1988, Proc. Natl. Acad. Sci. U.S.A. 85:7448-7451), etc.

Low stringency conditions are well known to those of skill in the art, and will vary predictably depending on the specific organisms from which the library and the labeled sequences are derived. For guidance regarding such conditions see, for example, Sambrook et al., 1989, Molecular Cloning, A Laboratory Manual (and periodic updates thereof), Cold Springs Harbor Press, N.Y.; and Ausubel et al., 1989, Current Protocols in Molecular Biology, Green Publishing Associates and Wiley Interscience, N.Y.

Alternatively, suitably labeled NHP nucleotide probes can be used to screen a human genomic library using appropriately stringent conditions or by PCR. The identification and characterization of human genomic clones is helpful for identifying polymorphisms (including, but not limited to, nucleotide repeats, microsatellite alleles, single nucleotide polymorphisms, or coding single nucleotide polymorphisms), determining the genomic structure of a given locus/allele, and designing diagnostic tests. For example, sequences derived from regions adjacent to the intron/exon boundaries of the human gene can be used to design primers for use in amplification assays to detect mutations within the exons, introns, splice sites (e.g., splice acceptor and/or donor sites), etc., that can be used in diagnostics and pharmacogenomics.

Further, a NHP homolog can be isolated from nucleic acid from an organism of interest by performing PCR using two degenerate or "wobble" oligonucleotide primer pools designed on the basis of amino acid sequences within the NHP products disclosed herein. The template for the reaction may be total RNA, mRNA, and/or cDNA obtained by reverse transcription of mRNA prepared from human or non-human cell lines or tissue known or suspected to express an allele of a NHP gene.

The PCR product can be subcloned and sequenced to ensure that the amplified sequences represent the sequence of the desired NHP gene. The PCR fragment can then be used to isolate a full length cDNA clone by a variety of methods. For example, the amplified fragment can be labeled and used to screen a cDNA library, such as a bacteriophage cDNA library. Alternatively, the labeled fragment can be used to isolate genomic clones via the screening of a genomic library.

PCR technology can also be used to isolate full length cDNA sequences. For example, RNA can be isolated, following standard procedures, from an appropriate cellular or tissue source (*i.e.*, one known, or suspected, to express a NHP gene, such as, for example, testis tissue). A reverse transcription (RT) reaction can be performed on the RNA using an oligonucleotide primer specific for the most 5' end of the amplified fragment for the priming of first strand synthesis. The resulting RNA/DNA hybrid may then be "tailed" using a standard terminal transferase reaction, the hybrid may be digested with RNase H, and second strand synthesis may then be primed with a complementary primer. Thus, cDNA sequences upstream of the amplified fragment can be isolated. For a review of cloning strategies that can be used, see *e.g.*, Sambrook *et al.,* 1989, supra.

A cDNA encoding a mutant NHP gene can be isolated, for example, by using PCR. In this case, the first cDNA strand may be synthesized by hybridizing an oligo-dT oligonucleotide to mRNA isolated from tissue known or suspected to be expressed in an individual putatively carrying a mutant NHP allele, and by extending the new strand with reverse transcriptase. The second strand of the cDNA is then synthesized using an oligonucleotide that hybridizes specifically to the 5' end of the normal gene. Using these two primers, the product is then amplified via PCR, optionally cloned into a suitable vector, and subjected to DNA sequence analysis through methods well known to those of skill in the art. By comparing the DNA sequence of the mutant NHP allele to that of a corresponding normal NHP allele, the mutation(s) responsible for the loss or alteration of function of the mutant NHP gene product can be ascertained.

Alternatively, a genomic library can be constructed using DNA obtained from an individual suspected of, or known to carry,a mutant NHP allele (e.g., a person manifesting a NHP-associated phenotype such as, for example, obesity, high blood pressure, connective tissue disorders, infertility, etc.), or a cDNA library can be constructed using RNA from a tissue known, or suspected, to express a mutant NHP allele. A normal NHP gene, or any suitable fragment thereof, can then be labeled and used as a probe to identify the corresponding mutant NHP allele in such libraries. Clones containing mutant NHP gene sequences can then be purified and subjected to sequence analysis according to methods well known to those skilled in the art.

Additionally, an expression library can be constructed utilizing cDNA synthesized from, for example, RNA isolated from a tissue known, or suspected, to express a mutant NHP allele in an individual suspected of or known to carry such a mutant allele. In this manner, gene products made by the putatively mutant tissue can be expressed and screened using standard antibody screening techniques in conjunction with antibodies raised against normal NHP product, as described below. (For screening techniques, see, for example, Harlow, E. and Lane, eds., 1988, "Antibodies: A Laboratory Manual", Cold Spring Harbor Press, Cold Spring Harbor NY).

Additionally, screening can be accomplished by screening with labeled NHP fusion proteins, such as, for example, alkaline phosphatase-NHP or NHP-alkaline phosphatase fusion proteins.

In cases where a NHP mutation results in an expressed gene product with altered function (*e.g.*, as a result of a missense or a frameshift mutation), polyclonal antibodies to NHP are likely to cross-react with a corresponding mutant NHP gene product. Library clones detected via their reaction with such labeled antibodies can be purified and subjected to sequence analysis according to methods well known in the art.

The invention also encompasses (a) DNA vectors that contain any of the foregoing NHP coding sequences and/or their complements (*i.e.*, antisense); (b) DNA expression vectors that contain any of the foregoing NHP coding sequences operatively associated with a regulatory element that directs the expression of the coding sequences (for example, baculo virus as described in U.S. Patent No. 5,869,336); (c) genetically engineered host cells that contain any of the foregoing NHP coding sequences operatively associated with a regulatory element that directs the expression of the coding sequences in the host cell; and (d) genetically engineered host cells that express an endogenous NHP gene under the control of an exogenously introduced regulatory element (*i.e.*, gene activation). As used herein, regulatory elements include, but are not limited to, inducible and non-inducible promoters, enhancers, operators and other elements known to those skilled in the art that drive and regulate expression. Such regulatory elements include but are not limited to the cytomegalovirus (hCMV) immediate early gene, regulatable, viral elements (particularly retroviral LTR promoters), the early or late promoters of SV40 adenovirus, the lac system, the *trp* system, the *TAC* system, the *TRC* system, the major operator and promoter regions of phage lambda, the control regions of fd coat protein, the promoter for 3-phosphoglycerate kinase (PGK), the promoters of acid phosphatase, and the promoters of the yeast α-mating factors.

The present invention also encompasses antibodies and anti-idiotypic antibodies (including Fab fragments), antagonists and agonists of a NHP, as well as compounds or nucleotide constructs that inhibit expression of a NHP gene (transcription factor inhibitors, antisense and ribozyme molecules, or gene or regulatory sequence replacement constructs), or promote the expression of a NHP (*e.g.*, expression constructs in which NHP coding sequences are operatively associated with expression control elements such as promoters, promoter/enhancers, etc.).

The NHPs or NHP peptides, NHP fusion proteins, NHP nucleotide sequences, antibodies, antagonists and agonists can be useful for the detection of mutant NHPs or inappropriately expressed NHPs for the diagnosis of disease. The NHP proteins or peptides, NHP fusion proteins, NHP nucleotide sequences, host cell expression systems, antibodies, antagonists, agonists and genetically engineered cells and non-human animals can be used for screening for drugs (or high throughput screening of combinatorial libraries) effective in the treatment of the symptomatic or phenotypic manifestations of perturbing the normal function of a NHP in the body. The use of engineered host cells and/or non-human animals may offer an advantage in that such systems allow not only for the identification of compounds that bind to the endogenous receptor for a NHP, but can also identify compounds that trigger NHP-mediated activities or pathways.

Finally, the NHP products can be used as therapeutics. For example, soluble derivatives such as NHP peptides/domains corresponding to NHP, NHP fusion protein products (especially NHP-Ig fusion proteins, *i.e.*, fusions of a NHP, or a domain of a NHP, to an IgFc), NHP antibodies and anti-idiotypic antibodies (including Fab fragments), antagonists or agonists (including compounds that modulate or act on downstream targets in a NHP-mediated pathway) can be used to directly treat diseases or disorders. For instance, the administration of an effective amount of soluble NHP, or a NHP-IgFc fusion protein or an anti-idiotypic antibody (or its Fab) that mimics the NHP could activate or effectively antagonize the endogenous NHP receptor. Nucleotide constructs encoding such NHP products can be used to genetically engineer host cells to express such products in vivo; these genetically engineered cells function as "bioreactors" in the body delivering a continuous supply of a NHP, a NHP peptide, or a NHP fusion protein to the body. Nucleotide constructs encoding functional NHP, mutant NHPs, as well as antisense and ribozyme molecules can also be used in "gene therapy" approaches for the modulation of NHP expression. Thus, the invention also discloses pharmaceutical formulations and methods for treating biological disorders.

Various aspects of the invention are described in greater detail in the subsections below.

### 5.1 THE NHP SEQUENCES

The cDNA sequences and the corresponding deduced amino acid sequences of the described NHPs are presented in the Sequence Listing. The NHP nucleotides were obtained from human cDNA libraries using probes and/or primers generated from human gene trapped sequence tags. Expression analysis has provided evidence that the described NHP can be expressed in human brain and testis cells as well as gene trapped human cells. The NHPs share sequence and structural similarity with methyltransferase proteins, and particularly catechol-O-methyltransferases from a variety of organisms. Similar methyltransferases have been shown to be associated with Parkinson's disease, development, and cancer. As such, methyltransferases have been widely studied as exemplified in U.S. Patent No. 6,001,607 which describes assays and applications for functionally related proteins, and polynucleotides encoding the same.

### 5.2 NHP AND NHP POLYPEPTIDES

NHPs, NHP polypeptides, NHP peptide fragments, mutated, truncated, or deleted forms of NHP, and/or NHP fusion proteins can be prepared for a variety of uses. These uses include but are not limited to the generation of antibodies, as reagents in diagnostic assays, the identification of other cellular gene products related to a NHP, as reagents in assays for screening for compounds that can be used as pharmaceutical reagents useful in the therapeutic treatment of mental, biological, or medical disorders and disease.

The Sequence Listing discloses the amino acid sequence encoded by the described NHP polynucleotides. The NHPs display initiator methionines in DNA sequence contexts consistent with translation initiation sites, and apparently display signal sequences which can indicate that the described NHP ORFs are secreted proteins or possibly membrane associated.

The NHP amino acid sequences of the invention include the amino acid sequences presented in the Sequence Listing as well as analogues and derivatives thereof, as well as any oligopeptide sequence of at least about 10-40, generally about 12-35, or about 16-30 amino acids in length first disclosed in the Sequence Listing. Further, corresponding NHP homologues from other species are encompassed by the invention. In fact, any NHP encoded by the NHP nucleotide sequences described above are within the scope of the invention, as are any novel polynucleotide sequences encoding all or any novel portion of an amino acid sequence presented in the Sequence Listing. The degenerate nature of the genetic code is well known, and, accordingly, each amino acid presented in the Sequence Listing, is generically representative of the well known nucleic acid "triplet" codon, or in many cases codons, that can encode the amino acid. As such, as contemplated herein, the amino acid sequences presented in the Sequence Listing, when taken together with the genetic code (see, for example, Table 4-1 at page 109 of "Molecular Cell Biology", 1986, J. Darnell et al. eds., Scientific American Books, New York, NY) are generically representative of all the various permutations and combinations of nucleic acid sequences that can encode such amino acid sequences.

The invention also encompasses proteins that are functionally equivalent to the NHPs encoded by the presently described nucleotide sequences as judged by any of a number of criteria, including, but not limited to, the ability to bind and cleave a substrate of a NHP, or the ability to effect an identical or complementary downstream pathway, or a change in cellular metabolism (*e.g.*, proteolytic activity, ion flux, tyrosine phosphorylation, etc.). Such functionally equivalent NHP proteins include, but are not limited to, additions or substitutions of amino acid residues within the amino acid sequence encoded by the NHP nucleotide sequences described above, but which result in a silent change, thus producing a functionally equivalent gene product. Amino acid substitutions can be made on the basis of similarity in polarity, charge, solubility, hydrophobicity, hydrophilicity, and/or the amphipathic nature of the residues involved. For example, nonpolar (hydrophobic) amino acids include alanine, leucine, isoleucine, valine, proline, phenylalanine, tryptophan, and methionine; polar neutral amino acids include glycine, serine, threonine, cysteine, tyrosine, asparagine, and glutamine; positively charged (basic) amino acids include arginine, lysine, and histidine; and negatively charged (acidic) amino acids include aspartic acid and glutamic acid.

A variety of host-expression vector systems can be used to express the NHP nucleotide sequences of the invention. Where, as in the present instance, the NHP products or NHP polypeptides can be produced in soluble or secreted forms (by removing one or more transmembrane domains where applicable), the peptide or polypeptide can be recovered from the culture media. Such expression systems also encompass engineered host cells that express a NHP, or a functional equivalent, *in situ.* Purification or enrichment of NHP from such expression systems can be accomplished using appropriate detergents and lipid micelles and methods well known to those skilled in the art. However, such engineered host cells themselves may be used in situations where it is important not only to retain the structural and functional characteristics of the NHP, but to assess biological activity, e.g., in drug screening assays.

The expression systems that may be used for purposes of the invention include, but are not limited to, microorganisms such as bacteria (*e.g., E. coli, B. subtilis)* transformed with recombinant bacteriophage DNA, plasmid DNA or cosmid DNA expression vectors containing NHP nucleotide sequences; yeast (*e.g., Saccharomyces, Pichia*) transformed with recombinant yeast expression vectors containing NHP encoding nucleotide sequences; insect cell systems infected with recombinant virus expression vectors (*e.g.,* baculovirus) containing NHP sequences; plant cell systems infected with recombinant virus expression vectors (*e.g.,* cauliflower mosaic virus, CaMV; tobacco mosaic virus, TMV) or transformed with recombinant plasmid expression vectors (*e.g.*, Ti plasmid) containing NHP nucleotide sequences; or mammalian cell systems (*e.g.,* COS, CHO, BHK, 293, 3T3) harboring recombinant expression constructs containing promoters derived from the genome of mammalian cells (e.g., metallothionein promoter) or from mammalian viruses (e.g., the adenovirus late promoter; the vaccinia virus 7.5K promoter).

In bacterial systems, a number of expression vectors may be advantageously selected depending upon the use intended for the NHP product being expressed. For example, when a large quantity of such a protein is to be produced for the generation of pharmaceutical compositions of or containing NHP, or for raising antibodies to a NHP, vectors that direct the expression of high levels of fusion protein products that are readily purified may be desirable. Such vectors include, but are not limited, to the E. coli expression vector pUR278 (Ruther et al., 1983, EMBO J. 2:1791), in which a NHP coding sequence may be ligated individually into the vector in frame with the *lacZ* coding region so that a fusion protein is produced; pIN vectors (Inouye & Inouye, 1985, Nucleic Acids Res. 13:3101-3109; Van Heeke & Schuster, 1989, J. Biol. Chem. 264:5503-5509); and the like. pGEX vectors (Pharmacia or American Type Culture Collection) can also be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. The PGEX vectors are designed to include thrombin or factor Xa protease cleavage sites so that the cloned target gene product can be released from the GST moiety.

In an insect system, *Autographa californica* nuclear polyhidrosis virus (AcNPV) is used as a vector to express foreign sequences. The virus grows in *Spodoptera frugiperda* cells. A NHP coding sequence can be cloned individually into non-essential regions (for example the polyhedrin gene) of the virus and placed under control of an AcNPV promoter (for example the polyhedrin promoter). Successful insertion of NHP coding sequence will result in inactivation of the polyhedrin gene and production of non-occluded recombinant virus (*i.e.*, virus lacking the proteinaceous coat coded for by the polyhedrin gene). These recombinant viruses are then used to infect Spodoptera frugiperda cells in which the inserted sequence is expressed (*e.g.*, see Smith et al., 1983, J. Virol. 46: 584; U.S. Patent No. 4,215,051).

In mammalian host cells, a number of viral-based expression systems may be utilized. In cases where an adenovirus is used as an expression vector, the NHP nucleotide sequence of interest may be ligated to an adenovirus transcription/translation control complex, e.g., the late promoter and tripartite leader sequence. This chimeric gene may then be inserted in the adenovirus genome by *in vitro* or *in vivo* recombination. Insertion in a non-essential region of the viral genome (*e.g.*, region E1 or E3) will result in a recombinant virus that is viable and capable of expressing a NHP product in infected hosts (*e.g.*, See Logan & Shenk, 1984, Proc. Natl. Acad. Sci. USA 81:3655-3659). Specific initiation signals may also be required for efficient translation of inserted NHP nucleotide sequences. These signals include the ATG initiation codon and adjacent sequences. In cases where an entire NHP gene or cDNA, including its own initiation codon and adjacent sequences, is inserted into the appropriate expression vector, no additional translational control signals may be needed. However, in cases where only a portion of a NHP coding sequence is inserted, exogenous translational control signals, including, perhaps, the ATG initiation codon, must be provided. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (See Bitter et al., 1987, Methods in Enzymol. 153:516-544).

In addition, a host cell strain may be chosen that modulates the expression of the inserted sequences, or modifies and processes the gene product in the specific fashion desired. Such modifications (*e.g.*, glycosylation) and processing (*e.g.*, cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the gene product may be used. Such mammalian host cells include, but are not limited to, CHO, VERO, BHK, HeLa, COS, MDCK, 293, 3T3, WI38, and in particular, human cell lines.

For long-term, high-yield production of recombinant proteins, stable expression is preferred. For example, cell lines which stably express the NHP sequences described above can be engineered. Rather than using expression vectors which contain viral origins of replication, host cells can be transformed with DNA controlled by appropriate expression control elements (*e.g.*, promoter, enhancer sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express the NHP product. Such engineered cell lines may be particularly useful in screening and evaluation of compounds that affect the endogenous activity of the NHP product.

A number of selection systems may be used, including but not limited to the herpes simplex virus thymidine kinase (Wigler, et al., 1977, Cell 11:223), hypoxanthine-guanine phosphoribosyltransferase (Szybalska & Szybalski, 1962, Proc. Natl. Acad. Sci. USA 48:2026), and adenine phosphoribosyltransferase (Lowy, et al., 1980, Cell 22:817) genes can be employed in tk⁻, hgprt⁻ or aprt⁻ cells, respectively. Also, antimetabolite resistance can be used as the basis of selection for the following genes: dhfr, which confers resistance to methotrexate (Wigler, et al., 1980, Natl. Acad. Sci. USA 77:3567; O'Hare, et al., 1981, Proc. Natl. Acad. Sci. USA 78:1527); gpt, which confers resistance to mycophenolic acid (Mulligan & Berg, 1981, Proc. Natl. Acad. Sci. USA 78:2072); neo, which confers resistance to the aminoglycoside G-418 (Colberre-Garapin, et al., 1981, J. Mol. Biol. 150:1); and hygro, which confers resistance to hygromycin (Santerre, et al., 1984, Gene 30:147).

Alternatively, any fusion protein can be readily purified by utilizing an antibody specific for the fusion protein being expressed. For example, a system described by Janknecht *et al.* allows for the ready purification of non-denatured fusion proteins expressed in human cell lines (Janknecht, et al., 1991, Proc. Natl. Acad. Sci. USA 88:8972-8976). In this system, the gene of interest is subcloned into a vaccinia recombination plasmid such that the gene's open reading frame is translationally fused to an amino-terminal tag consisting of six histidine residues. Extracts from cells infected with recombinant vaccinia virus are loaded onto Ni²⁺·nitriloacetic acid-agarose columns and histidine-tagged proteins are selectively eluted with imidazole-containing buffers.

Also encompassed by the present invention are fusion proteins that direct the NHP to a target organ and/or facilitate transport across the membrane into the cytosol. Conjugation of NHPs to antibody molecules or their Fab fragments could be used to target cells bearing a particular epitope. Attaching the appropriate signal sequence to the NHP would also transport the NHP to the desired location within the cell. Alternatively targeting of NHP or its nucleic acid sequence might be achieved using liposome or lipid complex based delivery systems. Such technologies are described in Liposomes; A Practical Approach, New,RRC ed., Oxford University Press, New York and in U.S. Patents Nos. 4,594,595, 5,459,127, 5,948,767 and 6,110,490 and their respective disclosures. Additionally embodied are novel protein constructs engineered in such a way that they facilitate transport of the NHP to the target site or desired organ, where they cross the cell membrane and/or the nucleus where the NHP can exert its functional activity. This goal may be achieved by coupling of the NHP to a cytokine or other ligand that provides targeting specificity, and/or to a protein transducing domain to facilitate passage across cellular membranes and can optionally be engineered to include nuclear localization sequences.

### 5.3 ANTIBODIES TO NHP PRODUCTS

Antibodies that specifically recognize one or more epitopes of a NHP, or epitopes of conserved variants of a NHP, or peptide fragments of a NHP are also disclosed. Such antibodies include but are not limited to polyclonal antibodies, monoclonal antibodies (mAbs), humanized or chimeric antibodies, single chain antibodies, Fab fragments, F(ab')₂ fragments, fragments produced by a Fab expression library, anti-idiotypic (anti-Id) antibodies, and epitope-binding fragments of any of the above.

The antibodies of the invention may be used, for example, in the detection of NHP in a biological sample and may, therefore, be utilized as part of a diagnostic or prognostic technique whereby patients may be tested for abnormal amounts of NHP. Such antibodies may also be utilized in conjunction with, for example, compound screening schemes for the evaluation of the effect of test compounds on expression and/or activity of a NHP gene product. Additionally, such antibodies can be used in conjunction with gene therapy to, for example, evaluate the normal and/or engineered NHP-expressing cells prior to their introduction into the patient. Such antibodies may additionally be used as a method for the inhibition of abnormal NHP activity. Thus, such antibodies may, therefore, be utilized as part of treatment methods.

For the production of antibodies, various host animals may be immunized by injection with the NHP, an NHP peptide (e.g., one corresponding to a functional domain of an NHP), truncated NHP polypeptides (NHP in which one or more domains have been deleted), functional equivalents of the NHP, or mutated variant of the NHP. Such host animals may include but are not limited to pigs, rabbits, mice, goats, and rats, to name but a few. Various adjuvants may be used to increase the immunological response, depending on the host species, including but not limited to Freund's adjuvant (complete and incomplete), mineral salts such as aluminum hydroxide or aluminum phosphate, surface active substances such as lysolecithin, pluronic polyols, polyanions, peptides, oil emulsions, and potentially useful human adjuvants such as BCG (bacille Calmette-Guerin) and *Corynebacterium parvum.* Alternatively, the immune response could be enhanced by combination and or coupling with molecules such as keyhole limpet hemocyanin, tetanus toxoid, diptheria toxoid, ovalbumin, cholera toxin or fragments thereof. Polyclonal antibodies are heterogeneous populations of antibody molecules derived from the sera of the immunized animals.

Monoclonal antibodies, which are homogeneous populations of antibodies to a particular antigen, can be obtained by any technique which provides for the production of antibody molecules by continuous cell lines in culture. These include, but are not limited to, the hybridoma technique of Kohler and Milstein, (1975, Nature 256:495-497; and U.S. Patent No. 4,376,110), the human B-cell hybridoma technique (Kosbor et al., 1983, Immunology Today 4:72; Cole et al., 1983, Proc. Natl. Acad. Sci. USA 80:2026-2030), and the EBV-hybridoma technique (1985, Monoclonal Antibodies And Cancer Therapy, Alan R. Liss, Inc., pp. 77-96). Such antibodies may be of any immunoglobulin class including IgG, IgM, IgE, IgA, IgD and any subclass thereof. The hybridoma producing the mAb of this invention may be cultivated in vitro or *in* vivo. Production of high titers of mAbs in vivo makes this the presently preferred method of production.

In addition, techniques developed for the production of "chimeric antibodies" (Morrison et al., 1984, Proc. Natl. Acad. Sci., 81:6851-6855; Neuberger et al., 1984, Nature, 312:604-608; Takeda et al., 1985, Nature, 314:452-454) by splicing the genes from a mouse antibody molecule of appropriate antigen specificity together with genes from a human antibody molecule of appropriate biological activity can be used. A chimeric antibody is a molecule in which different portions are derived from different animal species, such as those having a variable region derived from a murine mAb and a human immunoglobulin constant region. Such technologies are described in U.S. Patents Nos. 6,075,181 and 5,877,397 and their respective disclosures. Also encompassed by the present invention is the use of fully humanized monoclonal antibodies as described in US Patent No. 6,150,584 and respective disclosures.

Alternatively, techniques described for the production of single chain antibodies (U.S. Patent 4,946,778; Bird, 1988, Science 242:423-426; Huston et al., 1988, Proc. Natl. Acad. Sci. USA 85:5879-5883; and Ward et al., 1989, Nature 341:544-546) can be adapted to produce single chain antibodies against NHP gene products. Single chain antibodies are formed by linking the heavy and light chain fragments of the Fv region via an amino acid bridge, resulting in a single chain polypeptide.

Antibody fragments which recognize specific epitopes may be generated by known techniques. For example, such fragments include, but are not limited to: the F(ab')₂ fragments which can be produced by pepsin digestion of the antibody molecule and the Fab fragments which can be generated by reducing the disulfide bridges of the F(ab')₂ fragments. Alternatively, Fab expression libraries may be constructed (Huse et al., 1989, Science, 246:1275-1281) to allow rapid and easy identification of monoclonal Fab fragments with the desired specificity.

Antibodies to a NHP can, in turn, be utilized to generate anti-idiotype antibodies that "mimic" a given NHP, using techniques well known to those skilled in the art. (See, e.g., Greenspan & Bona, 1993, FASEB J 7(5):437-444; and Nissinoff, 1991, J. Immunol. 147(8):2429-2438). For example antibodies which bind to a NHP domain and competitively inhibit the binding of NHP to its cognate receptor can be used to generate anti-idiotypes that "mimic" the NHP and, therefore, bind and activate or neutralize a receptor. Such anti-idiotypic antibodies or Fab fragments of such anti-idiotypes can be used in therapeutic regimens involving a NHP signaling pathway.

## Claims

1. An isolated nucleic acid molecule encoding the amino acid sequence described in SEQ ID NO: 2.

2. An isolated nucleic acid molecule as claimed in claim 1 comprising the sequence disclosed in SEQ ID NO: 1.

3. An isolated nucleic acid molecule encoding the amino acid sequence described in SEQ ID NO:4.

4. An isolated peptide encoded by a nucleic acid as claimed in any one of claims 1 to 3.

5. An isolated nucleic acid molecule as claimed in any one of claims 1 to 3, said isolated nucleic acid molecule comprising a DNA vector.

6. A host cell genetically engineered to comprise a nucleic acid as claimed in any one of claims 1 to 4 or claim 5, and that is capable of expressing the peptide encoded by said nucleic acid, optionally on the surface of the cell.

7. A non-human transgenic animal genetically engineered to expresse a nucleic acid as claimed in any one of claims 1 t o 3 or claim 5.

8. Use of nucleic acid as claimed in any one of claims 1 to 3 or claim 5, a peptide as claimed claim 4, a host cell as claimed in claim 6, or a non-human animal as claimed in claim 7, in drug screening.

## Patentansprüche

1. Ein isoliertes Nukleinsäuremolekül, das für die Aminosäuresequenz kodiert, die in SEQ ID NO: 2 beschrieben ist.

2. Ein isoliertes Nukleinsäuremolekül, wie in Anspruch 1 beansprucht, aufweisend die Sequenz, die in SEQ ID NO: 1 offenbart ist.

3. Ein isoliertes Nukleinsäuremolekül, das für die Aminosäuresequenz kodiert, die in SEQ ID NO: 4 beschrieben ist.

4. Ein isoliertes Peptid, das durch eine Nukleinsäure kodiert wird, wie in einem der Ansprüche 1 bis 3 beschrieben.

5. Ein isoliertes Nukleinsäuremolekül, wie in einem der Ansprüche 1 bis 3 beansprucht, wobei das isolierte Nukleinsäuremolekül einen DNA-Vektor umfasst.

6. Eine Wirtszelle, die genetisch so verändert ist, dass sie eine Nukleinsäure umfasst, wie in einem der Ansprüche 1 bis 4 oder Anspruch 5 beansprucht, und die im Stande ist, das Peptid zu exprimieren, das durch die Nukleinsäure kodiert wird, gegebenenfalls auf der Oberfläche der Zelle.

7. Ein nicht-menschliches transgenes Tier, das genetisch so verändert ist, dass es eine Nukleinsäure wie in einem der Ansprüche 1 bis 3 oder Anspruch 5 beansprucht, exprimiert.

8. Verwendung einer Nukleinsäure wie in einem der Anspruche 1 bis 3 oder Anspruch 5 beansprucht, eines Peptids wie in Anspruch 4 beansprucht, einer Wirtszelle wie in Anspruch 6 beansprucht, oder eines nicht-menschlichen Tieres wie in Anspruch 7 beansprucht, zum Wirkstoffscreening.

## Revendications

1. Une molécule d'acide nucléique isolée qui code pour la séquence d'acide aminé décrite dans SEQ ID n° 2.

2. Une molécule d'acide nucléique isolée selon la revendication 1, comprenant la séquence décrite dans SEQ ID n° 1.

3. Une molécule d'acide nucléique isolée qui code pour la séquence d'acide aminé décrite dans SEQ ID n° 4.

4. Un peptide isolé codé par un acide aminé tel que revendiqué dans une quelconque des revendications 1 à 3.

5. Une molécule d'acide nucléique isolée tel que revendiqué dans une quelconque des revendications 1 à 3, ladite molécule d'acide nucléique isolée comprenant un vecteur ADN.

6. Une cellule hôte obtenue par ingénierie génétique comprenant un acide nucléique tel que revendiqué dans une quelconque des revendications 1 à 4 ou dans la revendication 5, et qui est capable d'exprimer le peptide codé par ledit acide nucléique, éventuellement sur la surface de la cellule.

7. Un animal transgénique non humain obtenu par ingénierie génétique pour exprimer un acide nucléique tel que revendiqué dans une quelconque des revendications 1 à 3 ou dans la revendication 5

8. Utilisation d'un acide nucléique tel que revendiqué dans une quelconque des revendications 1 à 3 ou dans la revendication 5, d'un peptide selon la revendication 4, d'une cellule hôte telle que revendiquée dans la revendications 6 ou d'un animal non humain tel que revendiqué la revendication 7 dans un criblage de médicament.


### SEQUENCE LISTING

<110> LEXICON GENETICS INCORPORATED
<120> Novel Human Enzymes and Polynucleotides Encoding the same
<130> 8717EP JSvn/TJH
<150> US 60/180,413
   <151> 2000-02-04
<160> 5
<170> FastSEQ for Windows version 4.0
<210> 1
   <211> 777
   <212> DNA
   <213> Homo sapiens
<400> 1
   atgtcccctg ccattgcatt ggccttcctg ccactggtgg taacattgct ggtgcggtac      60
   cggcactact tccgattgct ggtgcgcacg gtcttgctgc gaagcctccg agactgcctg     120
   tcagggctgc ggatcgagga gcgggccttc agctacgtgc tcacccatgc cctgcccggt     180
   gaccctggtc acatcctcac caccctggac cactggagca gccgctgcga gtacttgagc     240
   cacatggggc ctgtcaaagg tcagatcctg atgcggctgg tggaggagaa ggcccctgct     300
   tgtgtgctgg aattgggaac ctactgtgga tactctaccc tgcttattgc ccgagccctg     360
   ccccctgggg gtcgccttct tactgtggag cgggacccac gcacggcagc agtggctgaa     420
   aaactcatcc gcctggccgg ctttgatgag cacatggtgg agctcatcgt gggcagctca     480
   gaggacgtga tcccgtgcct acgcacccag tatcagctga gtcgggcaga cctggtgctc     540
   ctggcacacc ggccacgatg ttacctgagg gacctgcagc tgctggaggc ccatgcccta     600
   ctgccagcag gtgccaccgt gctggctgac catgtgctct tccctggtgc accccgcttc     660
   ttgcagtatg ctaagagctg tggccgctac cgctgccgcc tccaccacac tggccttcca     720
   gacttccctg ccatcaagga tggaatagct cagctcacct atgctggacc aggctga        777
<210> 2
   <211> 258
   <212> PRT
   <213> Homo sapiens
<400> 2
<210> 3
   <211> 507
   <212> DNA
   <213> Homo sapiens
<400> 3
   atgcggctgg tggaggagaa ggcccctgct tgtgtgctgg aattgggaac ctactgtgga    60
   tactctaccc tgcttattgc ccgagccctg ccccctgggg gtcgccttct tactgtggag   120
   cgggacccac gcacggcagc agtggctgaa aaactcatcc gcctggccgg ctttgatgag   180
   cacatggtgg agctcatcgt gggcagctca gaggacgtga tcccgtgcct acgcacccag   240
   tatcagctga gtcgggcaga cctggtgctc ctggcacacc ggccacgatg ttacctgagg   300
   gacctgcagc tgctggaggc ccatgcccta ctgccagcag gtgccaccgt gctggctgac   360
   catgtgctct tccctggtgc accccgcttc ttgcagtatg ctaagagctg tggccgctac   420
   cgctgccgcc tccaccacac tggccttcca gacttccctg ccatcaagga tggaatagct   480
   cagctcacct atgctggacc aggctga                                         507
<210> 4
   <211> 168
   <212> PRT
   <213> Homo sapiens
<400> 4
<210> 5
   <211> 2316
   <212> DNA
   <213> Homo sapiens
<400> 5
   agctcttact ctgcctcttg ttagctacgt gaccttgagc aaagcatgca tcctctgaac     60
   cttagcttct tcagaatgga aatcacaata ctgatcctga cttcttaggt tctgaggtca    120
   gaggaaatgt gagaacactc atgggaagct aagccaggac ctggcatgaa gtaagccaga    180
   tcctggtggg gtcttgactg ggagaacaat tccccccacc ctcacctcca gctcccccta    240
   tccccacaca gcctggttaa gtccaagctg aattcgcggc cgcttcaaat cccagttctg    300
   ctctgtgact ctggacaaaa gacttagcct ttctgagccg tggtttgtga aatataagga    360
   taataattgc tactggcaaa agctacacaa ataggcaaat tgtgggtatg ggattccctc    420
   cctacctccc tccaccccag ggcccaggta gggaccatgt cccctgccat tgcattggcc    480
   ttcctgccac tggtggtaac attgctggtg cggtaccggc actacttccg attgctggtg    540
   cgcacggtct tgctgcgaag cctccgagac tgcctgtcag ggctgcggat cgaggagcgg    600
   gccttcagct acgtgctcac ccatgccctg cccggtgacc ctggtcacat cctcaccacc    660
   ctggaccact ggagcagccg ctgcgagtac ttgagccaca tggggcctgt caaaggggac    720
   caggagggca gctggggcta tggtacaaga gacagatgag accccggctg gttgggagct    780
   gcagtgaggc aggtaggcat ttgagatatc ttttatcagg ggccctgcat ccatctccca    840
   tgtcttctgc aacagccatc tcccctcata ggtcagatcc tgatgcggct ggtggaggag    900
   aaggcccctg cttgtgtgct ggaattggga acctactgtg gatactctac cctgcttatt    960
   gcccgagccc tgccccctgg gggtcgcctt cttactgtgg agcgggaccc acgcacggca   1020
   gcagtggctg aaaaactcat ccgcctggcc ggctttgatg agcacatggt ggagctcatc   1080
   gtgggcagct cagaggacgt gatcccgtgc ctacgcaccc agtatcagct gagtcgggca   1140
   gacctggtgc tcctggcaca ccggccacga tgttacctga gggacctgca gctgctggag   1200
   gcccatgccc tactgccagc aggtgccacc gtgctggctg accatgtgct cttccctggt   1260
   gcaccccgct tcttgcagta tgctaagagc tgtggccgct accgctgccg cctccaccac   1320
   actggccttc cagacttccc tgccatcaag gatggaatag ctcagctcac ctatgctgga   1380
   ccaggctgag gtccaggccc aggggtactt actgatgccc acccccaccc ccacccaagc   1440
   agggacctca aaatcccctc cctttcctgt ttggggcctt gacacacgct gggctcaggg   1500
   ctagggagtc tctcttccca cctctgacct ctttcagcct ctacactgac ctcaagtgtc   1560
   aagttctatc aggctgcttg gtctcactag gccccctctt tccagagaga accatggact   1620
   gacagcaaga agcctgagct cccgacccag ctctgtcact gatttgctga gtgactccaa   1680
   gggaatcccc accttgctct gagatttaat cttctctctt aacacgaagg aagctggatg   1740
   ggagagctcc aggggcctcc cagttctcgg cctcagaaag cctcccatcc tcagcccatg   1800
   ccattctggg tgggatcaga ggaagtggca atgagttaga cgccctgcag gaatagctgg   1860
   atgcaagctg ggccagagaa aatggcacag aaccctggac ccagggccag ggatgccctg   1920
   gccttcccta actctggccc acctagccaa ttaggctttt acccagatct gagaaccaca   1980
   actgctctgg gtcagagaca ggacattcag aattagagca gagcctcggt ccactgcggc   2040
   ccccacacag gccccacctg ctagagccac tcacctctga ggctggcttg ccaataggaa   2100
   ccaggttgtt gtctttctcc gcgatgcttt ggagctgtgg gcaaaggcac agaggaacaa   2160
   ggccagagcc caagtagggc aggtcagggg catgggactg gcccattctg cccagaagac   2220
   aacccacacg tgttggggag aagcttcctc ccagttctca gggagataca atccctttct   2280
   tgtcatctgc catttatgaa cttgatccaa atactt                             2316